# EUROPÄISCHE PATENTANMELDUNG

(11) **EP 2 260 769 A1**
(43) Veröffentlichungstag der Anmeldung: **15.12.2010**
(21) Anmeldenummer: 10005799.1
(22) Anmeldetag: 04.06.2010
(51) Int. Cl.: A61B 17/00

(54) **Medizinisches Implantat zum Verschliessen von Gefässöffnungen**

(30) Priorität: 09.06.2009 DE 102009024390; 23.12.2009 DE 202009017418 U
(71) Anmelder: Bentley Surgical GmbH, 72379 Hechingen (DE)
(72) Erfinder: Eisold, Gerd, 72415 Groselfingen (DE)
(74) Vertreter: Kohler Schmid Möbus

(57) **Zusammenfassung**

Medizinisches Implantat zum Verschließen von Gefäßöffnungen und/oder Shunt-Verbindungen zwischen Körpergefäßen, das in einem kontrahierten Zustand minimal-invasiv positionierbar und in einen expandierten Zustand überführbar ist, indem es zwei voneinander beabstandete, im Durchmesser erweiterte Dichtelemente (11, 12; 11', 12'; 31, 32) aufweist, die die Gefäßöffnung oder Shunt-Verbindung auf beiden Seiten abdichten, wobei das Implantat (10, 10', 30) aus einem rohrförmigen Körper (20) durch Einbringen von Wandöffnungen (18, 19, 38) mindestens im Bereich der im expandierten Zustand einen erweiterten Durchmesser aufweisenden Dichtelemente (11, 12; 11', 12'; 31, 32) hergestellt ist.

## Beschreibung

Die Erfindung betrifft ein medizinisches Implantat zum Verschließen von Gefäßöffnungen und/oder Shunt-Verbindungen zwischen Körpergefäßen, das in einem kontrahierten Zustand minimal-invasiv positionierbar und in einen expandierten Zustand überführbar ist, indem es zwei voneinander beabstandete, im Durchmesser erweiterte Dichtelemente aufweist, die die Gefäßöffnung oder Shunt-Verbindung auf beiden Seiten abdichten.

Solche auch als Okkluder bezeichnete Implantate sind bereits bekannt. So beschreibt beispielsweise die EP 0 808 138 B1 einen Okkluder, der aus einem Metallgewebe aus miteinander verflochtenen Metalllitzen gefertigt ist. Der Okkluder wird mit Hilfe eines Katheters an die gewünschte Stelle im vaskulären System eines Patienten platziert und in den expandierten Zustand gebracht. Mit Hilfe des Okkluders lassen sich Gefäße eines Patienten verschließen, beispielsweise septale Defekte, wie etwa Defekte des Vorhofseptums. Auch ein persistierender Ductus (PDA) oder ein Ventrikelseptumdefekt können mit Okkludern behandelt werden, Ein weiteres, ähnliches Okklusionsinstrument ist aus der DE 10 2005 053 958 A1 bekannt. Auch dieses Okklusionsinstrument besteht aus einem Metallgewebe mit zwei schirmartigen Dichtelementen, wobei im implantierten Zustand eines der Dichtelemente auf der einen Seite der Gefäßöffnung und das andere Dichtelement auf der gegenüberliegenden Seite der Gefäßöffnung an der Gefäßwand anliegt und die zu schließende Gefäßöffnung abdichtet.

Die bekannten Okkluder haben den Nachteil, dass ihre Fertigung aus dünnen Metalldrähten oder Polymerfasern relativ aufwändig ist.

Der vorliegenden Erfindung liegt daher die Aufgabe zugrunde, ein Okklusionsimplantat zu schaffen, das einfach in der Herstellung und in der Handhabung ist.

Die Aufgabe wird mit einem medizinischen Implantat zum Verschließen von Gefäßöffnungen und/oder Shunt-Verbindungen zwischen Körpergefäßen gelöst, das in einem kontrahierten Zustand minimal-invasiv positionierbar und in einen expandierten Zustand überführbar ist, indem es zwei voneinander beabstandete, im Durchmesser erweiterte Dichtelemente aufweist, mit denen die Gefäßöffnung oder Shunt-Verbindung auf beiden Seiten abdichtbar ist, wobei das Implantat aus einem rohrförmigen Körper durch Einbringen von Wandöffnungen mindestens im Bereich der im expandierten Zustand einen erweiterten Durchmesser aufweisenden Dichtelemente hergestellt ist.

Die erfindungsgemäßen Implantate werden also nicht aus Geweben oder Netzen hergestellt, die zuvor in relativ aufwändigen Verfahren aus dünnen Fäden oder Drähten gefertigt werden. Stattdessen wird das Implantat aus einem massiven rohrförmigen Körper gefertigt, der zumindest an den Stehen, die im expandierten Zustand die Dichtelemente bilden, mit Wandöffnungen versehen wird. Durch das Einbringen der Wandöffnungen ist es möglich, die Dichtelemente mit den erweiterten Durchmessern herzustellen, beispielsweise indem der rohrförmige Körper gestaucht wird. Die Wandöffnungen lassen sich vorzugsweise durch Laserschneiden In den rohrförmigen Körper einbringen. Dieses Verfahren ist hochpräzise und relativ preisgünstig. Der gegenseitige Abstand der Wandöffnungen kann auch derart gewählt werden, dass die dazwischen stehen bleibenden Wandstege des rohrförmigen Körpers dicker sind als die Fäden oder Litzen der bekannten Okkluder. Dadurch lassen sich erfindungsgemäße Implantate mit einer höheren Formstabilität als die bekannten Okkluder herstellen.

Vorzugsweise können die Wandöffnungen dabei schlitzförmig ausgebildet sein. Diese Form der Wandöffnungen ermöglicht eine leichte Verformbarkeit des rohrförmigen Körpers, sodass die im expandierten Zustand einen erweiterten Durchmesser aufweisenden Dichtelemente einfach herstellbar sind. Weitere Vorteile ergeben sich, wenn mehrere, parallel zueinander ausgerichtete Wandöffnungen vorgesehen sind. Auch diese Maßnahme erleichtert die Verformbarkeit des Implantats. Der gegenseitige Abstand der Wandöffnungen und ihre Verteilung über den Umfang des rohrförmigen Körpers kann entsprechend den gewünschten Eigenschaften des Implantats gewählt werden. Die Konfiguration der Wandöffnungen bestimmt die Eigenschaften der Dichtelemente.

Bei einer bevorzugten Variante können sich die Wandöffnungen im Wesentlichen parallel zur Längsachse des Implantats erstrecken. Dadurch sind sie einfach in den rohrförmigen Körper einzubringen und gewährleisten außerdem ein leichtes Überführen des Implantats In den expandierten Zustand. Die Wandöffnungen können jedoch auch in einem spitzen Winkel zur Längsachse des Implantats verlaufen oder sich spiralförmig über den Umfang des Implantats erstrecken.

Weitere Vorteile ergeben sich, wenn das Implantat aus einer Formgedächtnislegierung, Insbesondere aus Nitinol gefertigt ist. Bei der Verwendung solcher Materialien lässt sich der expandierte Zustand des Implantats fixieren. Nitinol weist zugleich federnde Eigenschaften auf, sodass das Implantat zum Einführen in den Körper des Patienten in den kontrahierten Zustand gebracht und beispielsweise mittels eines Katheters eingeführt werden kann. Am Zielort kann der Katheter zurückgezogen werden, wodurch das Implantat die abgespeicherte expandierte Form annimmt, indem sie in der Lage ist, die Gefäßöffnung oder die Shunt-Verbindung abzudichten.

Alternativ zur Verwendung einer Formgedächtnisfegierung kann auch ein Formgedächtnispolymer zur Herstellung des erfindungsgemäßen Implantats verwendet werden. Polymere haben ebenfalls hervorragende Formgedächtniseigenschaften und lassen sich noch einfacher verformen als Nitinol.

Zur Erzielung einer flüssigkeitsdichten Abdichtung von Gefäßöffnungen können In oder auf den Dichtelementen flüssigkeitsdichte Membranen angeordnet sein. Diese Membranen werden mit Hilfe der Dichtelemente beidseits der Gefäßöffnungen positioniert und in dieser Lage gehalten. Die Membranen können aus unterschiedlichen Materialien gefertigt sein. Um eine hohe Körperverträglichkeit zu erzielen, ist jedoch ePTFE als Material für die Membranen zu bevorzugen.

Zur Herstellung des Implantats können Wandöffnungen in den rohrförmigen Körper nicht nur in den Bereichen der Dichtelemente, sondern auch in einem zentralen Bereich zwischen den Dichtelementen eingebracht werden. Wird das Implantat dergestalt ausgebildet, so ist es möglich, durch Stauchen und/oder Verwinden dieses zentralen Bereichs den Durchmesser des Implantats und den gegenseitigen Abstand der Dichtelemente zu verändern und/oder den zentrale Bereich in seinem Querschnitt dem Querschnitt der Gefäßöffnung oder der Shunt-Verbindung anzupassen. Damit lässt sich eine gute Zentrierung des Implantats in der Gefäßöffnung oder Shunt-Verbindung erzielen. Gleichzeitig werden die Dichtelemente gegen die Gefäßwand in Anlage gebracht, das heißt eine gute Dichtwirkung des Implantats erreicht.

Dabei können die schlitzförmigen Wandöffnungen des zentralen Bereichs in die schlitzförmigen Wandöffnungen im Bereich der Dichtelemente übergehen. Die Wandöffnungen können somit als durchgehende, sich bis zu den Endbereichen des Rohres erstreckende Schlitze gefertigt werden. Der Verzicht auf einen nicht geschlitzten Rohrabschnitt zwischen dem zentralen Bereich und den Dichtelementbereichen führt außerdem zu einer leichteren Verformbarkeit des zentralen Bereichs. Er kann damit einfacher an unterschiedliche Größen und Formen von Gefäßöffnungen oder Shunt-Verbindungen angepasst werden. Häufig weisen diese keinen runden, sondern einen ovalen oder schlitzförmigen Querschnitt auf. Aufgrund der flexiblen Gestaltung des zentralen Bereichs können jedoch mit einem einzigen Typ von Implantat eine ganze Reihe von Öffnungsgrößen und -formen abgedeckt werden. Die bisher erforderliche Bevorratung von Implantaten für unterschiedliche Durchmessergrößen der zu verschließenden Öffnungen im 1-Millimeter-Raster kann somit deutlich reduziert werden. Es können jetzt mit einem Implantat Durchmesserbereiche der Öffnungen von mehreren Millimetern versorgt werden.

Bei einer bevorzugten Ausgestaltung können die Wandöffnungen im zentralen Bereich parallel zur Längsachse des Implantats und im Bereich der beiden Dichtelemente spiralförmig verlaufen. Nach dem Überführen in den expandierten Zustand bilden in den Dichtelementbereichen die zwischen den Wandöffnungen verbleibenden Stege sich radial nach außen erstreckende Schlaufen. Der zentrale Bereich kann ebenfalls gestaucht werden, wodurch er eine bauchige Form annimmt. Durch Verdrehen um die Längsachse kann er außerdem in der Länge verkürzt werden. Der Umfang des zentralen Bereichs passt sich nach dem Positionieren des Implantats an der zu verschließenden Stelle dem Umfang der Gefäßöffnung oder der Shunt-Verbindung an.

Wie die bekannten Okkluder kann auch das erfindungsgemäße Implantat vorzugsweise mit Hilfe eines Katheters oder einer Wendel im Körper positionierbar sein. Zusätzlich kann das erfindungsgemäße Implantat durch einen Führungsdraht geführt und positioniert werden. Mit Hilfe des Katheters oder der Wendel kann das Implantat mit hoher Genauigkeit durch das vaskuläre System des Patienten bewegt und am gewünschten Einsatzort positioniert werden. Für die Befestigung des Katheters oder der Wendel kommen unterschiedliche Möglichkeiten in Betracht. Bei einer besonders einfachen Ausgestaltung kann der Katheter oder die Wendel mittels einer Schraubverbindung oder mittels eines Bajonettverschlusses mit einem der Enden des Implantats verbindbar sein.

Nach dem Einsetzen des Implantats soll dieses von Körpergewebe überwuchert werden. Dies lässt sich dadurch erleichtern, dass das gesamte Implantat eine biokompatible Beschichtung aufweisen kann. Nachfolgend werden bevorzugte Ausführungsbeispiele eines erfindungsgemäßen Implantats anhand der Zeichnung näher beschrieben.

Im Einzelnen zeigen:
- Fig. 1: eine Seitenansicht auf ein erfindungsgemäßes Implantat im expandierten Zustand;
- Fig. 2: eine Stirnansicht auf das Implantat aus Fig. 1;
- Fig. 3: eine perspektivische Ansicht eines rohrförmigen Körpers zur Herstellung eines erfindungsgemäßen Implantats;
- Fig. 4: eine perspektivische Ansicht eines rohrförmigen Körpers zur Herstellung eines dritten erfindungsgemäßen Implantats;
- Fig. 5: eine perspektivische Ansicht des dritten Implantats im expandierten Zustand;
- Fig. 6: eine Stirnansicht auf das dritte Implantat.

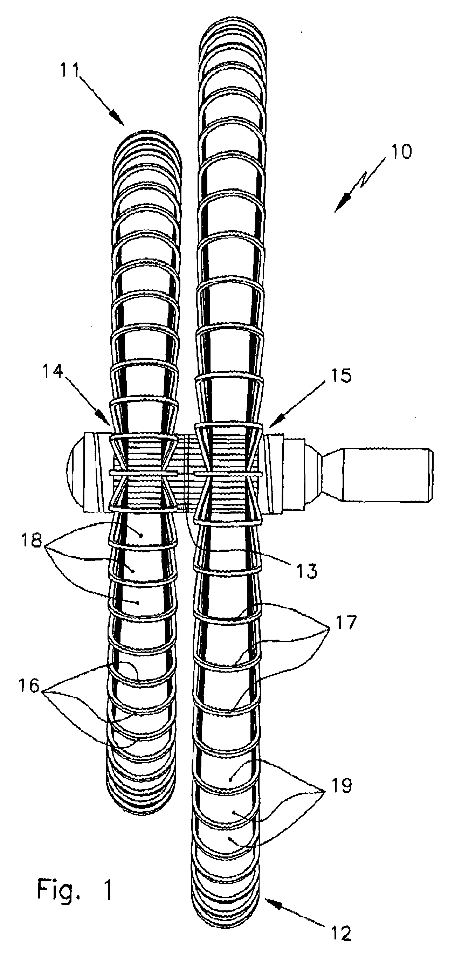

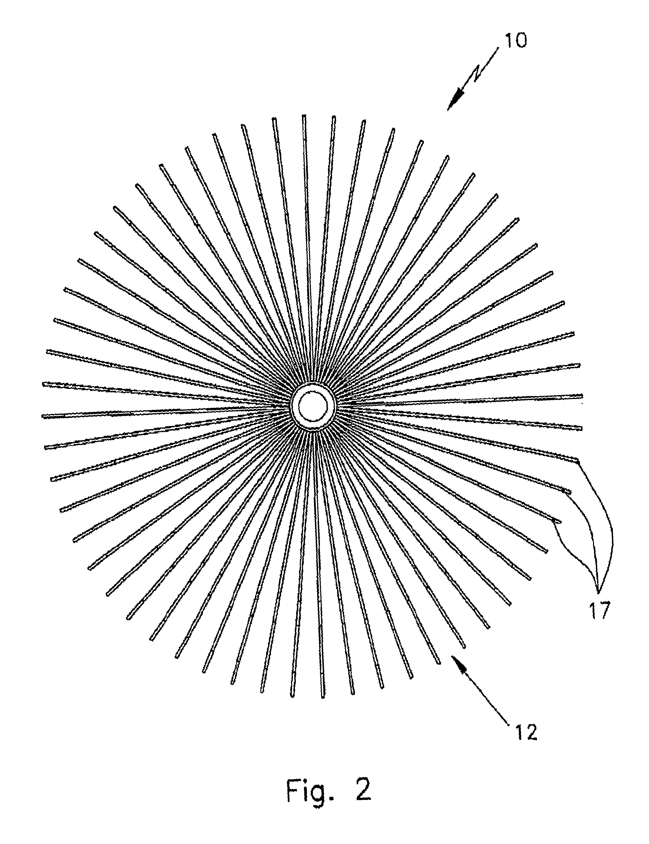

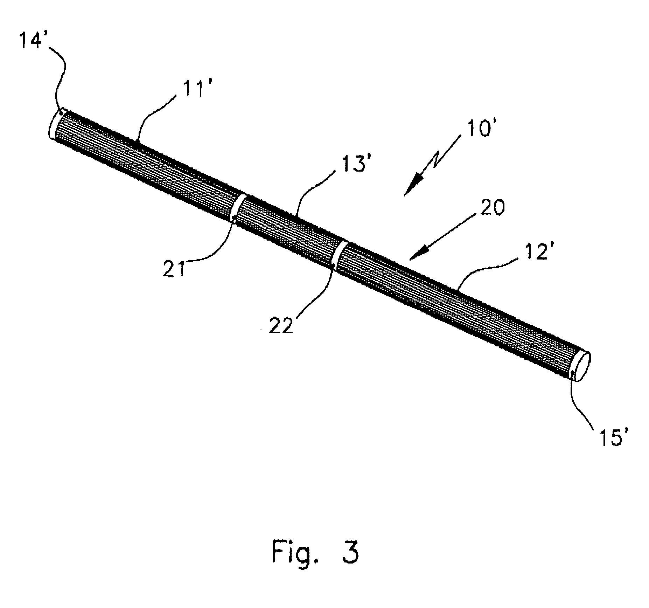

Das in Fig. 1 dargestellte Implantat 10 weist zwei Dichtelemente 11, 12 auf, die gegenüber einem dazwischen liegenden zentralen Bereich 13 sowie den Endbereichen 14, 15 einen deutlich erweiterten Durchmesser aufweisen. Im implantierten Zustand ragt der zentrale Bereich 13 durch die zu verschließende Gefäßöffnung bzw. Shunt-Verbindurig, während die beiden Dichtelemente 11, 12 beidseitig der Gefäßöffnung oder der Shunt-Verbindung an der Gefäßwand anliegen. Im Inneren oder an der Oberfläche der Dichtelemente 11, 12 sind hier nicht dargestellte flüssigkeitsdichte Membranen angeordnet, die dafür sorgen, dass das Implantat 10 die Gefäßöffnung oder die Shunt-Verbindung gegen den Durchtritt von Flüssigkeiten, insbesondere von Blut abdichtet. Das Implantat bestehend aus den Dichtelementen 11, 12, dem zentralen Bereich 13 sowie den beiden Endbereichen 14, 15 ist einteilig ausgebildet. Während die Bereiche 13, 14 und 15 von massiven Rohrabschnitten gebildet sind, werden die Dichtelemente 11, 12 von schlaufenförmigen Metallstegen 16, 17 gebildet, die gleichmäßig über den Umfang der Dichtelemente 11, 12 verteilt sind, wie insbesondere auch Fig. 2 zeigt, die das Implantat 10 in der Stirnansicht auf das Dichtelement 12 zeigt. Die Metallstege 16, 17 entstehen durch Einschneiden von schlitzförmigen Wandöffnungen 18, 19 in einen rohrförmigen Körper 20, wie er beispielsweise in Fig. 3 gezeigt ist. Durch Stauchen des rohrförmigen Körpers 20 in seiner Längsrichtung biegen sich dann die Metallstege 16, 17 zu Schlaufen auf, wodurch die Dichtelemente 11, 12 mit erweitertem Durchmesser entstehen. Dieser Zustand, der in Fig. 1 gezeigt ist, entspricht dem expandierten Zustand des Implantats 10. Wird das Implantat 10 dabei aus einem Material mit Formengedächtnis hergestellt, so kann diese expandierte Form im Material gespeichert werden.

Zum Einführen des Implantats 10 wird das Implantat 10 dagegen wieder in seinen kontrahierten Zustand gebracht, der demjenigen, der in Fig. 3 für ein leicht abgewandeltes Implantat 10' gezeigt ist, entspricht. Im kontrahierten Zustand sind die Implantate 10, 10' rohrförmig und lassen sich dadurch leicht mit einem Katheter oder einer Wendel minimal-invasiv über das vaskuläre System an den gewünschten Einsatzort verbringen. Ist das Implantat 10, 10' positioniert, so kann der expandierte, gespeicherte Zustand des Implantats 10, 10' aktiviert werden, wodurch das Implantat 10, 10' seine abdichtende Wirkung entfalten kann.

Das in Fig. 3 gezeigte Implantat 10' wird wie das Implantat 10 aus einem massiven rohrförmigen Körper 20 durch Einbringen von schlitzförmigen Wandöffnungen, insbesondere mit Hilfe eines Lasers hergestellt. Im Gegensatz zum Implantat 10 werden für das Implantat 10' jedoch nicht nur im Bereich der Dichtelemente 11', 12' schlitzförmige Wandöffnungen eingebracht, sondern auch im zentralen Bereich 13'. Als massive Rohrstücke bleiben nur die Endbereiche 14', 15' sowie die Übergänge 21, 22 zwischen den Dichtelementen 11', 12' und dem zentralen Bereich 13'.

Das Implantat 10' bietet im Gegensatz zum Implantat 10 die Möglichkeit, durch ein Verwinden des rohrförmigen Körpers 20 im zentralen Bereich 13' dort den Durchmesser des Implantats 10' zu verringern und gleichzeitig den gegenseitigen Abstand der Dichtelemente 11', 12' zu reduzieren. Damit ist das Implantat 10' in seinen Anpassungsmöglichkeiten an die im Gefäß vorhandene Situation flexibler als das Implantat 10, bei dem der Durchmesser des zentralen Bereichs 13 sowie der axiale Abstand der Dichtelemente 11 und 12 unveränderbar ist.

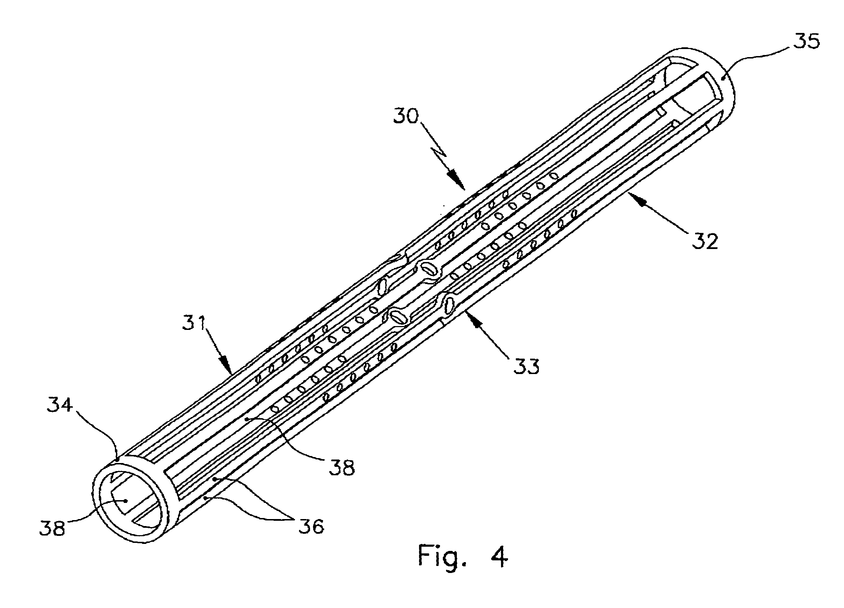

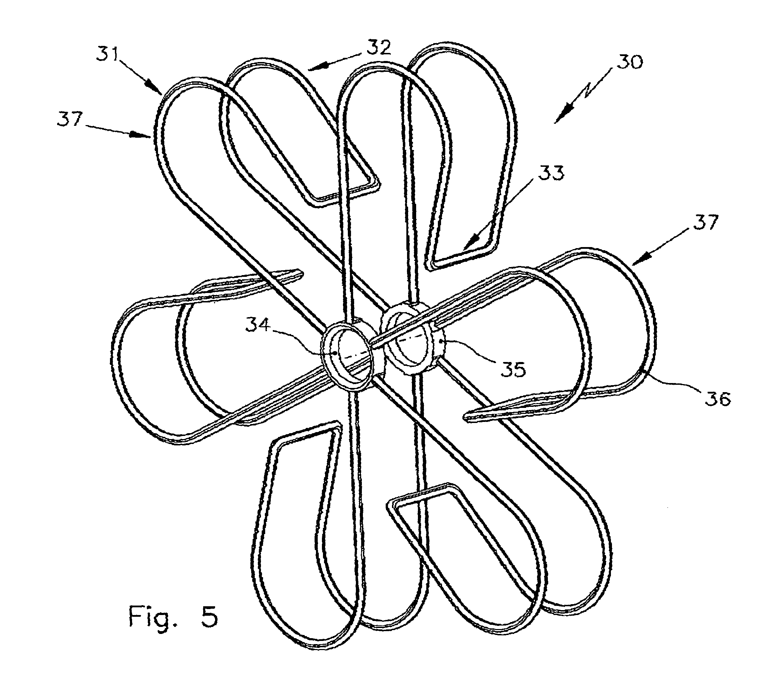

Fig. 4 zeigt einen rohrförmigen Körper zur Herstellung eines dritten Implantats 30, das im Gegensatz zum Implantat 10'sich über die beiden Dichtungsbereiche 31 und 32 und einen dazwischen liegenden zentralen Bereich 33 erstreckende Wandöffnungen 38 aufweist, die außerdem relativ breit sind, sodass dazwischen nur wenige Wandstege 36 verbleiben. Die Wandöffnungen 38 sind somit als durchgehende, sich bis zu den Endbereichen 34 und 35 des Rohres erstreckende Schlitze gefertigt. Der Verzicht auf nicht geschlitzte Rohrabschnitte zwischen dem zentralen Bereich 33 und den Dichtelementbereichen 31 und 32 führt zu einer noch leichteren Verformbarkeit des zentralen Bereichs 33 im Vergleich zum zentralen Bereich 13' des Implantats 10'. Er lässt sich damit einfach an unterschledliche Größen und Formen von Gefäßöffnungen oder Shunt-Verbindungen anpassen. Dies verdeutlicht Fig. 5, die das Implantat 30 im expandierten Zustand zeigt. Die Wandstege 36 bilden im Bereich der Dichtelemente 31 und 32 radial nach außen ragende Schlaufen 37. Im zentralen Bereich 33 verlaufen die Wandstege 36 parallel zueinander, sind radial jedoch nach außen gedrückt worden, sodass sie im vergleich zu den Rohrenden 34, 35 einen deutlich größeren Durchmesser zwischen sich einschließen.

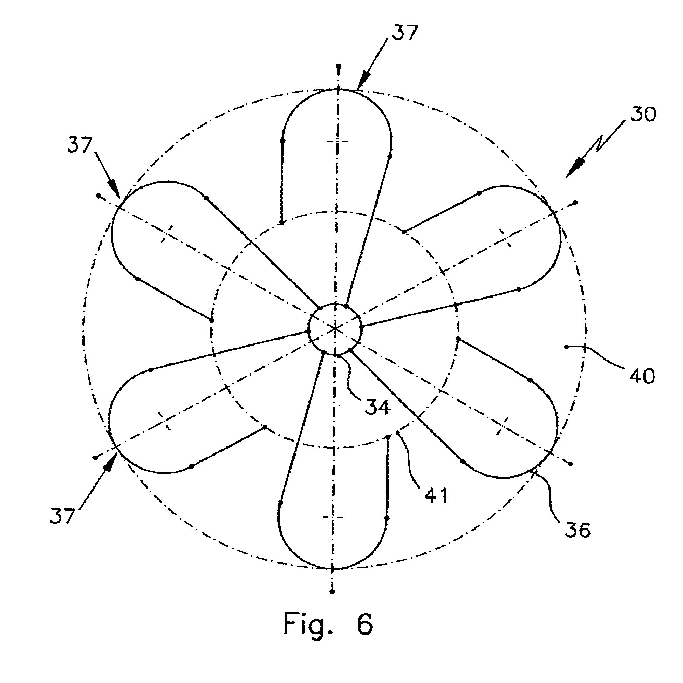

In Fig. 6 ist die Stirnansicht des Implantats 30 dargestellt, aus der auch die Anordnung einer strichpunktiert eingezeichneten Membran 40 im Dichtbereich 31 deutlich wird. Die Membran 40 aus einem flüssigkeitsdichten Material kann zischen den beiden Hälften der radialen Schlaufen 37 aufgespannt und beispielsweise an den äußeren Bögen der Schlaufen 37 befestigt werden. Sie ist damit in der Lage, eine Gefäßöffnung, deren Durchmesser in etwa dem Durchmesser des Kreises 41 entspricht, sicher zu verschließen. Auf dem Kreis 41 liegen die Abschnitte der Wandstege 36 (Fig. 5) Im zentralen Bereich 33 des Implantats 30, der nach dem Einsetzen des Implantats 30 durch die Gefäßöffnung hindurchragt. Auch im Dichtbereich 32 wird eine Membran 40 angeordnet, sodass die Gefäßöffnung von beiden Seiten her abgedichtet ist. Es versteht sich, dass die Implantate 10' und 30 auch in Gefäßöffnungen oder Shurit-Verbindungen eingesetzt werden können, die keinen kreisrunden, sondern einen eher ovalen oder schlitzförmigen Querschnitt aufweisen. Durch ein entsprechendes Verformen der Wandstege 36 im zentralen Bereich 13'. 33 können diese auch bei solchen Gefäßöffnungen in Anlage an den Rand der Öffnung gebracht werden und dadurch das Implantat 10', 30 zentrieren.

Die Implantate 10, 10' und 30 können beispielsweise aus Nitinol oder einer anderen Metalllegierung mit Formengedächtnis oder aber auch aus einem Formgedächtnispolymer hergestellt sein. Die Membranen 40 Im Bereich der Dichtelemente 11, 12; 11', 12', 31, 32 können vorzugsweise aus ePTFE gefertigt sein, da sich dieses Material durch eine sehr gute Körperverträglichkeit auszeichnet.

Die in den Figuren gezeigten Implantate 10, 10', 30 wurden durch Einbringen von schlitzförmigen Wandöffnungen 18, 19, 38 hergestellt, die parallel zur Längsachse des rohrförmigen Körpers 20 verlaufen. Dies ist jedoch nicht zwingend. Die Öffnungen 18, 19, 38 können mit der Längsachse des rohrförmigen Körpers 20 auch einen Winkel bilden, wodurch auch die Stege 16, 17, 36 Schlaufen bilden, deren Anfangs- und Endpunkte in tangentialer Richtung versetzt zueinander sind. Auch spiralförmig verlaufende Wandöffnungen 18, 19 oder andere Verläufe sind möglich.

## Patentansprüche

1. Medizinisches Implantat zum Verschließen von Gefäßöffnungen und/oder Shunt-Verbindungen zwischen Körpergefäßen, das in einem kontrahierten Zustand minimal-invasiv positionierbar und in einen expandierten Zustand überführbar ist, indem es zwei voneinander beabstandete, im Durchmesser erweiterte Dichtelemente (11, 12; 11', 12'; 31, 32) aufweist, die die Gefäßöffnung oder Shunt-Verbindung auf beiden Seiten abdichten, **dadurch gekennzeichnet, dass** das Implantat (10, 10', 30) aus einem rohrförmigen Körper (20) durch Einbringen von Wandöffnungen (18, 19, 38) mindestens im Bereich der im expandierten Zustand einen erweiterten Durchmesser aufweisenden Dichtelemente (11, 12; 11', 12'; 31, 32) hergestellt ist.

2. Implantat nach Anspruch 1, **dadurch gekennzeichnet, dass** die Wandöffnungen (18, 19, 38) schlitzförmig ausgebildet sind.

3. Implantat nach Anspruch 1 oder 2, **dadurch gekennzeichnet, dass** mehrere, parallel zueinander ausgerichtete Wandöffnungen (18, 19, 38) vorgesehen sind.

4. Implantat nach einem der vorhergehenden Ansprüche, **dadurch gekennzeichnet, dass** sich die Wandöffnungen (18, 19, 38) im Wesentlichen parallel zur Längsachse des Implantats (10, 10', 30) erstrecken.

5. Implantat nach einem der Ansprüche 1 bis 3, **dadurch gekennzeichnet, dass** die Wandöffnungen (18, 19, 38) in einem spitzen Winkel zur Längsachse des Implantats (10, 10', 30) angeordnet sind.

6. Implantat nach einem der Ansprüche 1 bis 3, **dadurch gekennzeichnet, dass** sich die Wandöffnungen (18, 19, 38) spiralförmig über den Umfang des Implantats (10, 10', 30) erstrecken.

7. Implantat nach einem der vorhergehenden Ansprüche, **dadurch gekennzeichnet, dass** auch in einem zwischen den Dichtelementen (11, 12; 11', 12', 31, 32) liegenden zentralen Bereich (13, 13', 33) schlitzförmige Wandöffnungen (18, 19, 38) vorgesehen sind.

8. Implantat nach Anspruch 7, **dadurch gekennzeichnet, dass** die schlitzförmigen Wandöffnungen (38) des zentralen Bereichs (33) in die schlitzförmigen Wandöffnungen (38) im Bereich der Dichtelemente (31, 32) übergehen.

9. Implantat nach Anspruch 7 oder 8, **dadurch gekennzeichnet, dass** die Wandöffnungen (18, 19, 38) im zentralen Bereich (13', 33) parallel zur Längsachse des Implantats (10', 30) und im Bereich der beiden Dichtelemente (11', 12', 31, 32) spiralförmig verlaufen.

10. Implantat nach einem der vorhergehenden Ansprüche, **dadurch gekennzeichnet, dass** es aus dem expandierten Zustand durch eine reversible elastische oder pseudoelastische Verformung in den kontrahierten Zustand überführbar ist.

11. Implantat nach einem der vorhergehenden Ansprüche, **dadurch gekennzeichnet, dass** es aus einer Formgedächtnislegierung, insbesondere aus Nitinol, oder aus einem Formgedächtnispolymer gefertigt ist.

12. Implantat nach einem der vorhergehenden Ansprüche, **dadurch gekennzeichnet, dass** in oder auf den Dichtelementen (11, 12; 11', 12'; 31, 32) flüssigkeitsdichte Membranen (40) angeordnet sind.

13. Implantat nach Anspruch 12, **dadurch gekennzeichnet, dass** die Membranen (40) aus ePTFE gefertigt sind.

14. Implantat nach einem der Ansprüche 7 bis 13, **dadurch gekennzeichnet, dass** durch Stauchen und/oder Verwinden des zentralen Bereichs (13', 33) die beiden Dichtelemente (11', 12', 31, 32) gegen die die Gefäßöffnung oder Shunt-Verbindung begrenzende Gefäßwand in Anlage bringbar sind und/oder der zentrale Bereich (13', 33) in seinem Querschnitt dem Querschnitt der Gefäßöffnung oder der Shunt-Verbindung anpassbar ist.

15. Implantat nach einem der vorhergehenden Ansprüche, **dadurch gekennzeichnet, dass** es mit Hilfe eines Katheters oder einer Wendel im Körper positionierbar ist.

16. Implantat nach Anspruch 15, **dadurch gekennzeichnet, dass** der Katheter oder die Wendel an mindestens einem der Enden (14, 15; 14', 15') des Implantats (10, 10') befestigbar ist.

17. Implantat nach Anspruch 16, **dadurch gekennzeichnet, dass** der Katheter oder die Wendel in eines der rohrförmigen Enden (14, 15; 14', 15') des Implantats (10,10') einschraubbar oder mittels eines Bajonettverschlusses befestigbar ist.
